# EUROPEAN PATENT SPECIFICATION

(11) **EP 3 781 700 B1**
(45) Date of publication and mention of the grant of the patent: **11.02.2026**
(21) Application number: 19719356.8
(22) Date of filing: 18.04.2019
(51) Int. Cl.: C12Q 1/02, G01N 33/58, G01N 21/64, C12Q 1/18, C12Q 1/10

(54) **DETECTING CELL VITALITY**
NACHWEIS DER ZELLVITALITÄT
DÉTECTION DE LA VITALITÉ CELLULAIRE

(30) Priority: 19.04.2018 GB 201806419; 26.10.2018 GB 201817435
(43) Date of publication of application: 24.02.2021
(73) Proprietor: The University Of Warwick, Coventry CV4 8UW (GB)
(72) Inventor: STRATFORD, James Peter, Coventry CV4 7AL (GB); ASARI, Munehiro, Coventry CV4 7AL (GB)
(74) Representative: Barker Brettell LLP
(86) International application number: PCT/GB2019/051103
(87) International publication number: WO 2019/202327

(56) References cited:
- EP-A1- 0 143 901
- EP-A1- 2 913 391
- WO-A2-2006/058185
- JP-A- 2012 005 381
- US-A1- 2004 115 614
- US-A1- 2008 044 879
- ADENOCARCINOMAS MUCINOUS ET AL: "% of stroma in tumor samples Differentiation level in tumor samples Supplemental Figure S1: Histopathological characterization of the tumor samples", 23 July 2012 (2012-07-23), XP055592022, Retrieved from the Internet <URL:http://clincancerres.aacrjournals.org/content/clincanres/suppl/2012/07/23/1078-0432.CCR-12-0372.DC1/fig1.pdf> [retrieved on 20190527]
- S. JULIEN ET AL: "Characterization of a Large Panel of Patient-Derived Tumor Xenografts Representing the Clinical Heterogeneity of Human Colorectal Cancer", CLINICAL CANCER RESEARCH, vol. 18, no. 19, 23 July 2012 (2012-07-23), pages 5314 - 5328, XP055591886, ISSN: 1078-0432, DOI: 10.1158/1078-0432.CCR-12-0372
- XUEWEN ZHOU ET AL: "Electrokinetically controlled fluid injection into unicellular microalgae", ELECTROPHORESIS, vol. 38, no. 20, 1 October 2017 (2017-10-01), pages 2587 - 2591, XP055599047, ISSN: 0173-0835, DOI: 10.1002/elps.201600548
- ROTH B L ET AL: "BACTERIAL VIABILITY AND ANTIBIOTIC SUSCEPTIBILITY TESTING WITH SYTOX GREEN NUCLEIC ACID STAIN", APPLIED AND ENVIRONMENTAL MICROBIOLOGY, AMERICAN SOCIETY FOR MICROBIOLOGY, US, vol. 63, no. 6, 1 January 1997 (1997-01-01), pages 2421 - 2431, XP000929110, ISSN: 0099-2240

## Description

### Field of the Invention

The invention relates to detecting cell vitality, i.e. determining whether cells are alive (vital) or dead (non-vital).

### Background

Conventional methods of detecting the presence of vital, or viable, cells in a sample generally involves culturing the sample in a medium to determine whether, and to what extent, the cells reproduce. This type of test is typical for detecting the presence and quantity of bacteria in a sample. The requirement to culture a sample involves significant delays to allow a sample to be cultured, which may be of the order of days before a result is available.

Some fluorescent dyes, such as the Sytox^{®} family of nucleic acid stains, can be used to detect cells with compromised membranes. This is because the dye is hydrophilic or charged and has no route by which it would ordinarily cross the lipid membrane. Cells with non-specific pores, such as those created by electroporation, will provide a path by which the dye can cross the membrane, bind to DNA and consequently produce a fluorescent signal. Cells with intact membranes, however, produce no signal, and these dyes cannot be used to detect vital cells. An indication of cell vitality can therefore only be made by discounting disintegrated cells.

Zuewen Zhou et al, in "Electrokinetically controlled fluid injection into unicellular microalgae", Electrophoresis 2017, 38, 2587-2591, disclose electrokinetically controlled microinjection of motile cells with a microinjection system using glass capillary pipettes. The viability of impaled cells was confirmed for more than an hour under 0.01V using the fluorescein diacetate/propidium iodide dual fluorescent dye based assay.

WO 2006/058185 A2 discloses a device and method for electroporation-based delivery of molecules into cells and dynamic monitoring of cell responses, including devices and methods for transfecting a cell or cell population and dynamic monitoring of cellular events. A variety of microelectronic devices are provided that incorporate functions such as electroporation, modulation of a transmembrane potential and dynamic monitoring of cellular functions and mechanisms.

### Summary of the Invention

According to a first aspect of the invention, there is provided a method of detecting vitality of cells in a sample, comprising:
providing a sample comprising cells and a fluorescent dye that responds to a change in membrane polarization;
disposing a portion of the sample containing a medium within which is provided the cells and the fluorescent dye in a test volume between a pair of electrodes;
applying a voltage across the pair of electrodes to generate an electric field across the portion of the sample;
illuminating the test volume;
measuring a fluorescence response from the test volume over a period of time after applying the voltage; and
detecting cells in the sample to be vital dependent on a change in their fluorescence response over the period of time.

The change in fluorescence response may for example be an increase or a decrease in response over the period of time, depending on the type of fluorescent dye used to detect the cellular membrane potentials.

The method addresses the problem of how to rapidly and reliably detect vital cells in a sample. The method is capable of detecting such cells in a much shorter time, and directly on the sample in question, compared with conventional techniques involving culturing.

The electric field in typical applications may be less than 1 kV/cm. The aim in this case is not to open pores in the cell membrane, as with electroporation, but instead to preferentially accelerate introduction of the fluorescent dye for vital cells but not for non-vital cells. The electric field may in typical applications be greater than 100 V/cm, with an example range between around 100 V/cm and 800 V/cm or between around 400 and 800 V/cm. The method preferably does not involve electroporation of the cells. In other words, the electric field is preferably selected such that electroporation of the cells does not occur while the voltage is applied.

The electric field may be alternating, for example applied in the form of one or more pulses, so that electrolysis of the sample is reduced or avoided. The voltage may be applied for only a short period such as less than 10s or less than 5s. A minimum time period over which the voltage is applied may be as short as 0.1s.

The method may further comprise the step of detecting cells in the sample to be non-vital cells if their fluorescence response decreases or does not rise over the period of time.

The period of time over which detection is carried out may for example be up to 30 minutes, or in some cases may be less than 60s or 30s, and may be less than 10s, and optionally greater than 0.1s.

The fluorescent dye may be a substance which produces a fluorescent response that indicates a changing electrical field or ion gradient across a cellular membrane. This may be a Nernstian dye, for example a flavin, such as thioflavin T. The dye does not, however, need to be Nernstian, and may instead be a substance that can travel or change its conformation as a result of membrane potential changes in response to voltage stimulation.

**In** some implementations, the sample may flow through the test volume from a first location to a second location of the test volume over the period of time, the steps of illuminating the test volume with light and measuring a fluorescent response being repeated at the first and second locations.

The step of detecting may comprise detecting cells in the sample to be vital if their fluorescence response increases, for example by more than a factor of two, over the period of time.

The method preferably relates to detection of vitality or viability of bacterial cells, i.e. the ability of such cells to proliferate. The method may therefore be used to detect a strain of bacteria in a sample, by first treating the sample with an antibiotic, and then performing the method to detect bacteria resistant to the antibiotic. The bacteria may for example be a coliform such as *Escherichia coli,* which is resistant to certain antibiotics including penicillin and vancomycin.

### Detailed Description

The invention is described in further detail below by way of example and with reference to the accompanying drawings, in which:
figure 1 is a schematic diagram of an example apparatus for measuring the vitality of cells in a sample;
figure 2 is a schematic plan view of an array of interdigitated electrodes for use in the apparatus of figure 1;
figure 3 is a micrograph of a sample of cells after application of an electric field in the presence of a fluorescent dye;
figures 4a, 4b and 4c are images of fluorescence from cells at different times during the application of an electric field;
figure 5 is a plot of relative fluorescence response over time during the application of an electric field for two selections of cells in a sample;
figure 6 is a further plot of relative fluorescence response over time during the application of an electric field for two selections of cells in a sample;
figure 7 is a schematic diagram of an alternative example apparatus for measuring the vitality of cells in a sample;
figure 8 is a schematic diagram of a further alternative example apparatus for measuring the vitality of cells in a sample;
figure 9 is a schematic flow diagram illustrating an example method for detecting vitality of cells in a sample according to the invention;
figure 10 is a schematic diagram of an example apparatus for measuring the vitality of cells in a sample;
figure 11 is a schematic diagram of a circuit arrangement for the apparatus of figure 10;
figure 12 is a schematic sectional view of an example optical assembly for the apparatus of figure 10;
figure 13 is a plan view of an example electrode layout for a sample holder;
figure 14 is a plan view of a portion of the example electrode layout of figure 13; and
figure 15 is a schematic diagram of an arrangement of a sample holder with the electrode layout of figure 13.

The change in membrane potential by an external electrical field (*Δψ*) is quantitatively described by the Schwan equation: *Δψ =* 1.5 r *E* cos*θ*. Here, r is the radius of an idealized spherical cell, *E* is the field strength of external electrical field, and *θ* is the angle to the electrical field.

According to this equation, an external electrical field can open voltage-gated potassium channels by altering the transmembrane potential of a cell. The Schwan equation enables a coarse estimation that the field strength required to cause the gating of a voltage-sensitive channel on a bacterial membrane is approximately in the range of 400 ~ 800 V/cm.

Opening of potassium channel lead to hyperpolarization of the cellular membrane when cells are vital because vital cells store a large amount of potassium in the cytoplasm. **In** non-vital cells, metabolism is limited or non-existent so there is no metabolic energy available to restore the potassium ion gradient. This means the membrane potential collapses, i.e. depolarises, when potassium channels open upon stimulation by an electrical pulse. The addition of a fluorescent dye that responds to a change in membrane polarization is therefore used to determine whether cells are vital depending on how the dye responds to the cell membrane being subject to an external electric field. Hyperpolarization may therefore be shown as a fluorescence increase when a Nernstian dye is used, or may be shown as a decrease in fluorescence signal for other types of dyes. A fluorescent dye such as FluoVolt^{®}, for example, decreases its fluorescence signal when hyperpolarized. Other fluorescence indicators for membrane potential may change their spectra according to membrane polarity, such as di-4-ANEPPS dyes.

Although the primary mechanism involved in introducing molecules is considered to be the opening of selective ion channels due to an applied electric field, other mechanisms may be operating. For example, an electrical pulse may temporarily change the ion concentration in the extracellular medium, changing the ion gradient across the cell membrane and causing efflux, such that there is no change in transporter state but a change in gradient that drives transport. The response could also be in part be due to the induction of stress in the cells, which can also trigger ion channel opening, especially potassium channels. This stress could be due to the electrical signal or chemical substances generated electrochemically during application of an electrical pulse. The response of the cells could also be in part due to the production of reactive species by an electrochemical reaction caused by the flow of electrical current between the electrodes. These species may modify the channels or trigger a stress response. Such species may be reactive oxygen species or oxidising chlorine compounds. All of the above described mechanisms have the common property that they involve modifying the conductivity of ion channels in the cell membrane or the rate of transport of ions, and consequently the movement of a molecule into the cell.

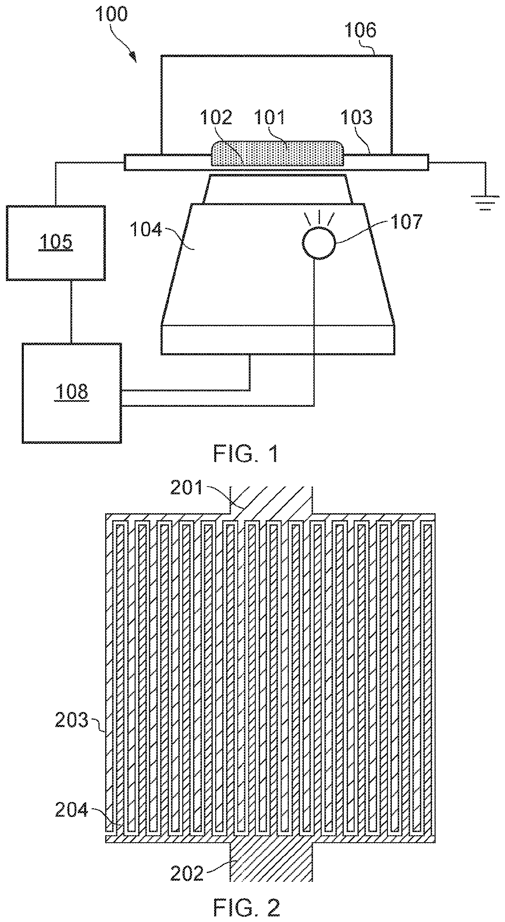

Figure 1 illustrates an example apparatus 100 for measuring the vitality of cells in a sample. The sample 101 is placed within a sample chamber 102 on the surface of a substrate 103, on to which electrodes (not shown in figure 1) have been applied. The sample 101 contains a medium within which is provided cells and a fluorescent dye that may be absorbed through the cell membranes. The medium may for example be in the form of a liquid or gel medium.

Optics 104 are provided adjacent the substrate 102, the optics 104 comprising a light source 107 arranged to direct light on to the sample 101 and a light detector arranged to detect light fluorescing from the sample 101 in response. In the illustrated example the light source 107 is positioned on the same side as the optics for viewing the sample 101. The light source may alternatively be positioned on the opposite side to the optics. The light source may comprise a ring of sources, such as LEDs or lamps, arranged in a ring or above the sample. The angle of the incident light in each case is preferably such that an angle of incidence of light on to the sample 101 is sufficiently low to avoid direct entry of light into the optics for detecting fluorescence. This minimises direct illumination of the detector by the fluorescent excitation light source.

A voltage generator 105 is connected to the electrodes on the substrate 103, for providing a controllable electrical field across portions of the sample 101. An example of the type of electrodes that may be provided on the surface of the substrate 103 is illustrated in figure 2. The electrodes 201, 202 are interdigitated, providing regions between adjacent opposing fingers 203, 204 of the electrodes within which a controllable electric field may be applied to a portion of the sample 101 placed on the surface of the substrate 103 over the electrodes 201, 202.

A controller 108 is connected to the voltage generator 105, light source 107 and optics 104 and configured to control the voltage generator 105 and acquire images from the optics 104 over a defined period of time a voltage signal is applied to the sample 101. The controller 108 may for example acquire a first image at the onset of the period of time before the voltage signal is applied, then a second image at the end of the period of time the signal is applied, and calculate a third image based on a difference between the first and second images, such as differences in luminance levels, outputting the third image to determine cells that show a change in fluorescence over the period of time.

To make the sample chamber, an array of interdigitated or evenly spaced signal and ground electrodes, which may for example be made of Ti-Au, indium tin oxide (ITO) or another conductive material, may be deposited onto a glass or other transparent hard substrate by a deposition technique such as physical or chemical vapour deposition or by screen printing.

The substrate 103 may be of a type allowing clear optical imaging at high resolution using common microscope objectives of a high numerical aperture from the reverse side of the transparent substrate, as shown in the arrangement in figure 1. This means the substrate will typically be optically clear and thinner than 0.2mm. Multiple sets of electrodes may be deposited on the substrate 103 to allow multiple samples to be analysed either consecutively or simultaneously.

External circuitry such as the voltage supply 105 may be connected to the substrate 103 using contact pads and standard electrical connectors.

Spacing between the electrodes will typically be less than 100 microns, with a preferred spacing of around 50 microns. A common spacing may be used across multiple electrode pairs so that a constant electric field can be applied with the same voltage. Using this range of electrode spacing, an electrical field of around 60 V/mm can be applied without the total voltage applied between the electrodes causing electrolysis of the sample.

The substrate 103 may be encased in a gas and/or moisture tight housing 106. The housing 106 may be transparent to allow light to pass through the sample from above and below. The light source may be provided on either side of the substrate in figure 1.

This voltage source 105 may be configured to generate electrical signals as well as recording these signals, and may be arranged to selectively apply and monitor voltage signals on multiple sets of electrodes on the substrate, allowing voltages supplied to different samples to be controlled.

**In** an experimental setup, bacterial cells were inoculated on agarose pads and placed on the electrode surface, enabling monitoring of cells at single-cell resolution by microscopy. The membrane potential dynamics of cells was monitored using the fluorescent Nernstian indicator dye, Thioflavin T (ThT), which has been used extensively with bacteria.

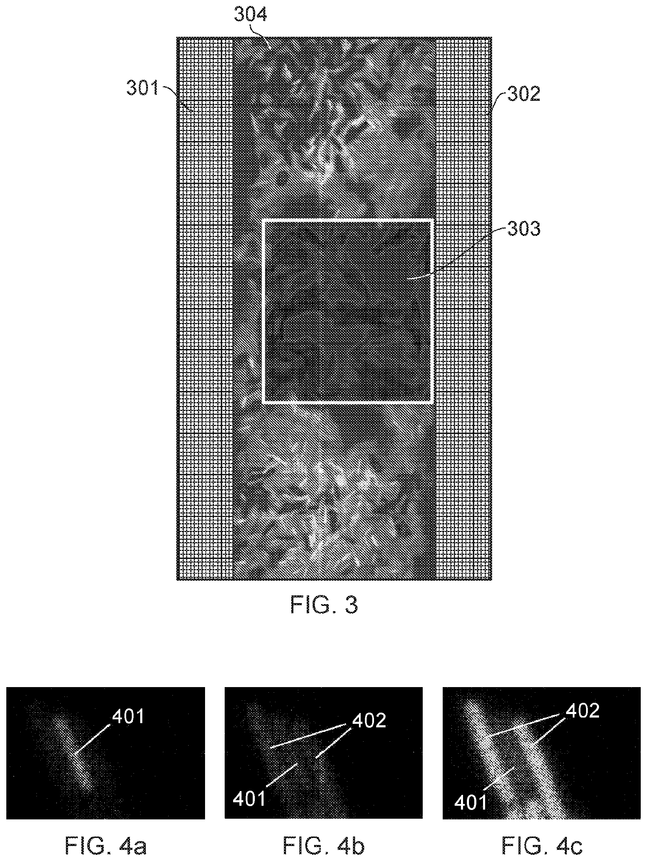

When a series of electrical pulses (±1.5 V AC 0.1 kHz for 2.5 seconds) were applied to *E. coli* (K12 strain) cells placed in the electrode gaps, the pulses caused hyperpolarization (seen by an increase of ThT fluorescence signal) in vital cells. Figure 3 illustrates the effect of applying an electric field across a sample 304 as viewed between a pair of opposing electrodes 301, 302 in an apparatus of the type illustrated in figure 1. Before the application of an electric field, a portion of the sample was subjected to ultraviolet radiation sufficient to kill cells within the defined region 303 of the sample holder. An electric field was then applied, and the image shown represents the difference in fluorescence over a period of time after the application of the electric field. As can be seen, the change in fluorescence in the region 303 that was subjected to UV radiation can be distinguished from the change in fluorescence for the rest of the sample 304. Whereas the region of the sample unaffected by UV radiation showed an increase in fluorescence, the region 303 affected by UV did not. This provides a clear indication that the change in fluorescence is an indication of the vitality of cells within the sample.

Figures 4a to 4c shows microscope images at different times during the application of an electric field to a sample of *Bacillus subtilis.* A signal of 3V in amplitude was applied to the sample held between electrodes with a spacing of 50 µm, giving a peak electrical field strength of between 400 and 800 V/cm. This compares with electroporation of bacteria, which may use a minimum of 3.000-24,000 V/cm. Figure 4a shows the sample within 1 second of the electric field being applied, showing a single bacterium 401 fluorescing. Figure 4b shows the sample after a further second, and two further bacteria 402 can now be seen to be fluorescing, while the strength of fluorescence of the first bacterium 401 decreases. Figure 4c shows the sample after a further two seconds, and the two further bacteria are now strongly fluorescing while fluorescence from the first bacterium has remained low. This example shows that a measure of vitality of cells can be obtained in a matter of seconds, demonstrating that the period of time the electric field needs to be applied before a measure of vitality is possible may be less than 10 seconds, and could be less than 5 seconds or even less than 3 seconds.

Living cells accumulate a Nernstian dye to much higher levels than the surrounding medium. This process is selective for Nernstian dyes which can already cross the cell membrane and excludes dyes such as Sytox^{®}, which do not pass through living cell membrane. The process does not cause transport of the dye by electroporation of the cells, which is only achieved at considerably higher electric field levels, but instead through enhancement of transport across a living cell membrane with an existing cell potential.

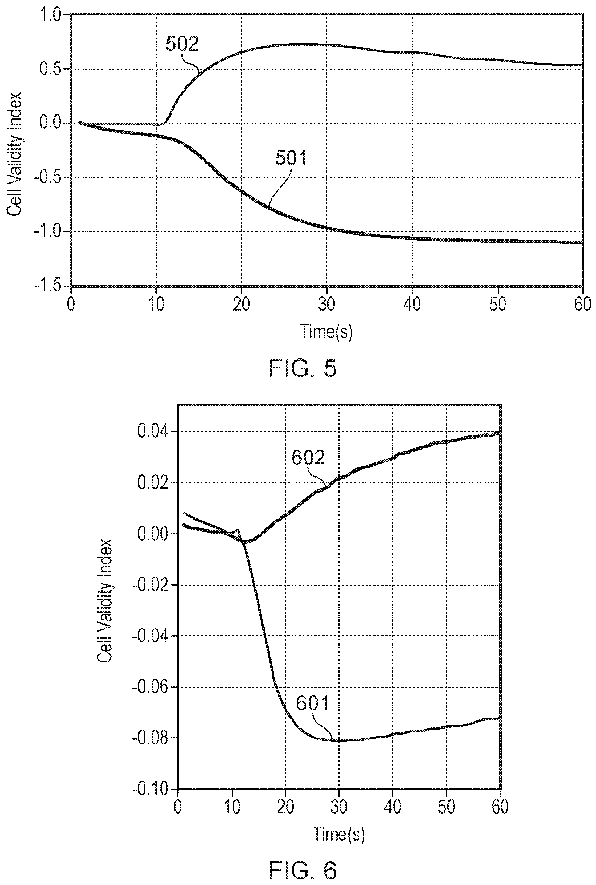

Figure 5 shows the results of subjecting a sample of *Bacillus subtilis* to selective UV radiation treatment and subsequent treatment by the application of an electric field. The sample (shown in figure 3) was subjected to alternating 3V pulses at a frequency of 100Hz for 2.5 seconds. The mean response 501 of ten UV-treated cells is shown in figure 5 alongside the mean response 502 of ten non-UV-treated cells. A cell validity index, being a logarithmic scale of the ratio between current and initial fluorescence shows a relative increase or decrease in fluorescence, indicating clearly the difference in response between live and dead cells. Live cells in this example showed a substantial increase, whereas dead cells showed a substantial decrease.

Figure 6 shows the log of a fluorescent response of the two cells types, *B. subtilis* and *E. coli* in the presence of vancomycin. Vancomycin is inactive against coliforms such as *E. coli,* but targets almost all other types of bacteria. In the mixed culture. While vancomycin treated *B. subtilis* cells showed depolarization 601, *E. coli* cells were hyperpolarized 602. This demonstrates that the process can selectively show the vitality of coliforms in a mixed culture. The presence of *E. coli* or other coliforms could therefore be confirmed through fluorescence analysis of a sample treated with a selective antibiotic such as vancomycin.

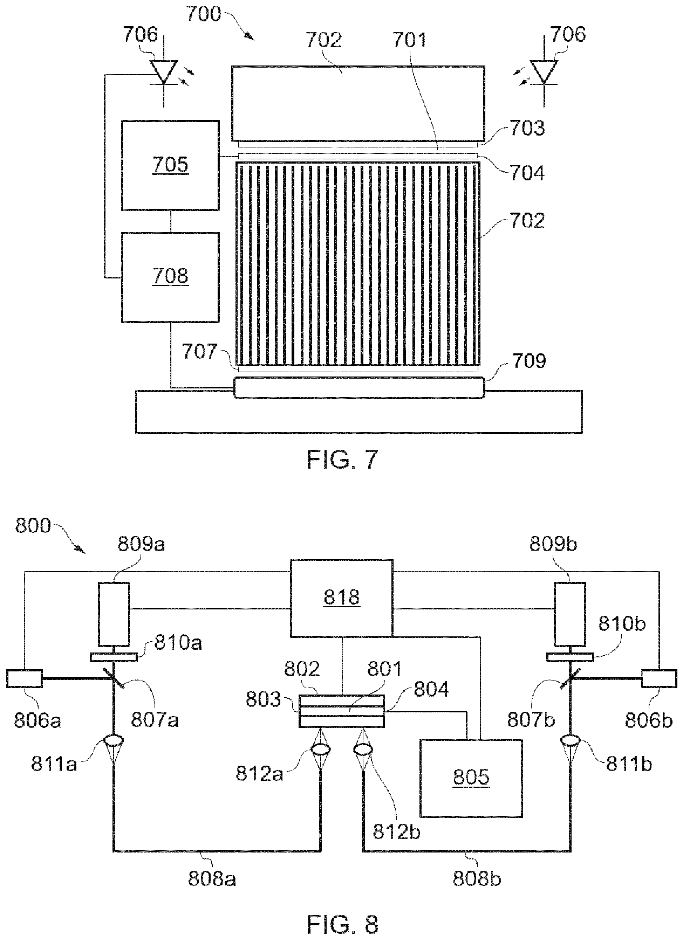

Figure 7 shows a further example of an apparatus 700 for detecting vitality of cells in a sample. **In** this case the apparatus comprises a test volume 701 for containing a sample between a transparent lid 702 and an optical fibre plate 702. Electrodes are provided on one or both of the surfaces 703, 704 of the lid 702 and optical fibre plate 702 defining opposing faces of the test volume 701. The electrodes may for example be in the form of pairs of electrodes on one of the plates, an interdigitated electrode pattern, or may be provided on both plates in the form of transparent electrodes, where the gap between the plates defines the electric field across the sample.

A voltage generator 705 is connected to the electrodes, and a light source 706 arranged to illuminate the test volume 701 through the lid 702. The light source 706, two of which are shown in figure 7, may be arranged to illuminate the test volume 701 at a shallow angle, for example at less than 45° to the surfaces 703, 704. The effect of this is to avoid light passing directly into the optic fibre plate 702 due to the numerical aperture of the plate 702 limiting the angle at which light can be passed into the fibres of the plate 702.

A light detector 709 is provided on an opposing side of the optical fibre plate 702. The light detector 709 is in the form of an imaging sensor such as a CCD. Interposing the optical fibre plate 702 between the imaging sensor 709 and the test volume 701 allows the sensor 709 to directly image the cells within the test volume without the need for intervening lens arrangements.

A filter 707 may be provided between the imaging sensor 709 and the test volume 701, in this case between the imaging sensor 709 and an adjacent face of the optical fibre plate 702. The filter 707 may selectively filter out a range of wavelengths of light including that emitted by the light source 706. The light source may for example emit blue light, and the filter 707 may allow transmission of green light to allow fluorescence from the test volume to be passed to the imaging sensor 709.

A controller 708 is connected to the voltage generator 705, light source 706 and imaging sensor 709 and configured to control the voltage generator 705 and acquire images from the imaging sensor 709 over a defined period of time a voltage signal is applied to the sample 701. The controller 708 may for example acquire a first image at the onset of the period of time before the voltage signal is applied, then a second image at the end of the period of time the signal is applied, and calculate a third image based on a difference between the first and second images, such as differences in luminance levels, outputting the third image to determine cells that show a change in fluorescence over the period of time.

Further details of example apparatus and experimental results for *B. subtilis* and *E. coli* are provided in GB application 1817435.9, to which this application claims priority.

Figure 8 illustrates a further alternative apparatus 800 for detecting the vitality of cells in a sample. The apparatus comprises a sample holder 801 in the form of a flow channel in a microfluidic device 802, arranged to flow a sample from an inlet 803 to an outlet 804. The channel 801 may be sized to allow cells to flow through the device 802 one at a time so that fluorescence measurements can be taken on individual cells. A pair of electrodes may be provided on opposing walls of the flow channel, the electrodes connected to a voltage generator 805.

The apparatus 800 comprises two sets of light sources and light detectors. A first light source 806a transmits (excitation) light to illuminate a first part of the flow channel 801, the light being transmitted via a dichromic reflector 807a and into an optical fibre 808a via a collimating lens 811a to the flow channel 801 via a further lens 812a. Fluorescence emission light from the flow channel 801 at a different wavelength is transmitted back along the optical fibre 808a and through the dichromic reflector to a light detector 809a via a filter 810a.

A second light source 806b transmits light to illuminate a second part of the flow channel 801 downstream from the first part, the light again being transmitted via a dichromic reflector 807b and into an optical fibre 808b via a collimating lens 811b to the flow channel 801 via a further lens 812b. Fluorescence light from the flow channel 801 at a different wavelength is transmitted back along the optical fibre 808b and through the dichromic reflector to a light detector 809b via a filter 810b.

Excitation light may for example be provided by a 405 nm laser diode, and a beam focused onto a 10-20um spot on the flow channel 801 in the microfluidic device 802. If a cell is present, fluorescence light is emitted which retraces the path of the excitation light back through the fibre towards the source, but instead of being reflected into the source, passes the dichroic mirror to the photodetector 809a. Two identical setups are used to quantify cell fluorescence by allowing a comparison between fluorescence as a cell passes the first part of the flow channel and then as the same cell passes the second part of the flow channel. Knowing the speed at which the cells pass through the flow channel allows a measure of relative change in fluorescence to be determined on a cell-by-cell basis. Each light detector 809a, 809b may for example be a photomultiplier tube or an avalanche photodiode.

A controller 818 is connected to the voltage generator 805, light sources 806a, 806b and detectors 809a, 809b and configured to control the voltage generator 805 and acquire signals from the detectors 809a, 809b over a defined period of time a voltage signal is applied to a sample flowing through the device 802. The controller 808 may for example store successive readings of fluorescence from the detectors 809a, 809b at time intervals corresponding to a time taken for a part of the sample to flow between the first and second parts of the flow channel 801, and output a difference between these readings that corresponds to the change in fluorescence.

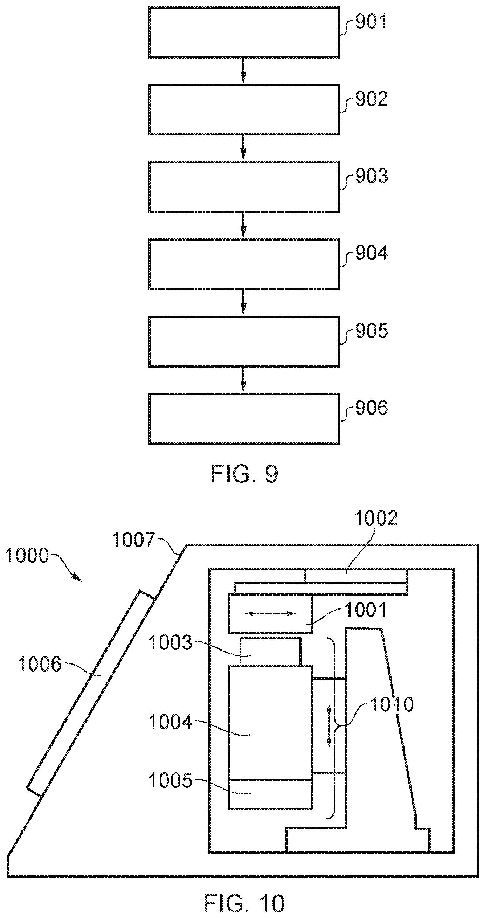

Figure 9 illustrates a schematic flow diagram showing a method of detecting vitality of cells in a sample. The method starts by providing a sample comprising cells and a fluorescent dye (step 901). The sample, or a portion thereof, is disposed in a test volume between a pair of electrodes (step 902). A voltage is then applied (step 903) between the pair of electrodes to generate an electric field across the portion of the sample in the test volume. The test volume is illuminated (step 904) and a fluorescence response is measured (step 905) over a period of time after applying the voltage across the electrodes. Cells in the sample are then detected as being vital (step 906) if their fluorescence response increases over the period of time. At least steps 903 to 906 may be automated and performed by a suitably programmed controller.

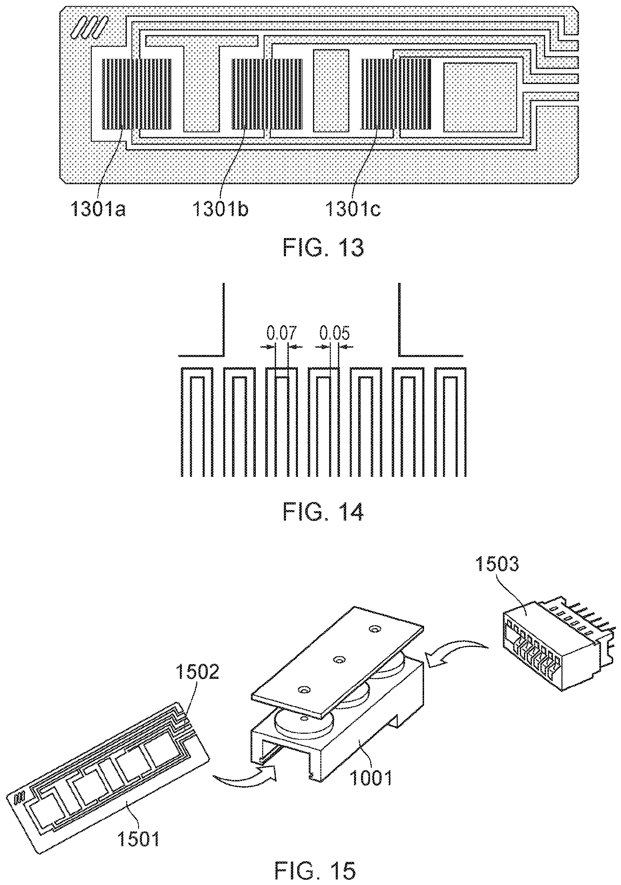

Figure 10 illustrates an example apparatus 1000 for measuring the vitality of cells in a sample. The sample is contained in a holder 1001 mounted on a translation stage 1002, which allows the sample holder 1001 to be moved in two directions in an x-y plane as with a conventional microscope translation stage. The stage 1002 preferably allows for movement of a sample slide (not shown) attached to the holder 1001 to allow sequential readings to be taken in two or more individual positions on the slide. An example slide 1501 and holder 1001 are shown in further detail in figure 15, the slide 1501 in this example having three locations where a sample may be deposited. The slide 1501 inserts into the holder 1001 and comprises an electrode layout with edge contacts 1502 for attaching to a PCB edge connector 1503.

Referring again to figure 10, the apparatus 1000 comprises an optical assembly 1010 having a lens assembly 1003 and image sensor 1004, which are translatable relative to the sample holder 1001 in the z direction, i.e. orthogonal to the x-y plane across which the sample holder 1001 is translatable. Translation of the optical assembly 1010 allows for focusing of the image sensor 1004 on to the sample in the sample holder 1001. The image sensor may comprise a CMOS, CCD or other type of sensor for providing an electrical signal from a spatially structure light input. A controller 1005, which may be part of, or separate from, the optical assembly 1010, comprises a microcontroller and a board level computer, which controls electrical stimulation to the sample through the edge connector 1503 and controls the image sensor 1004, processing image data to provide a required output signal, as described above and in further detail in GB1817435.9. An interface with the controller 1005 may be provided via a screen 1006 on a casing 1007 of the apparatus 1000, which contains all of the above mentioned components. The screen 1006 may for example be a touch screen to allow a user to control the apparatus and to display data, including bacterial cell counts.

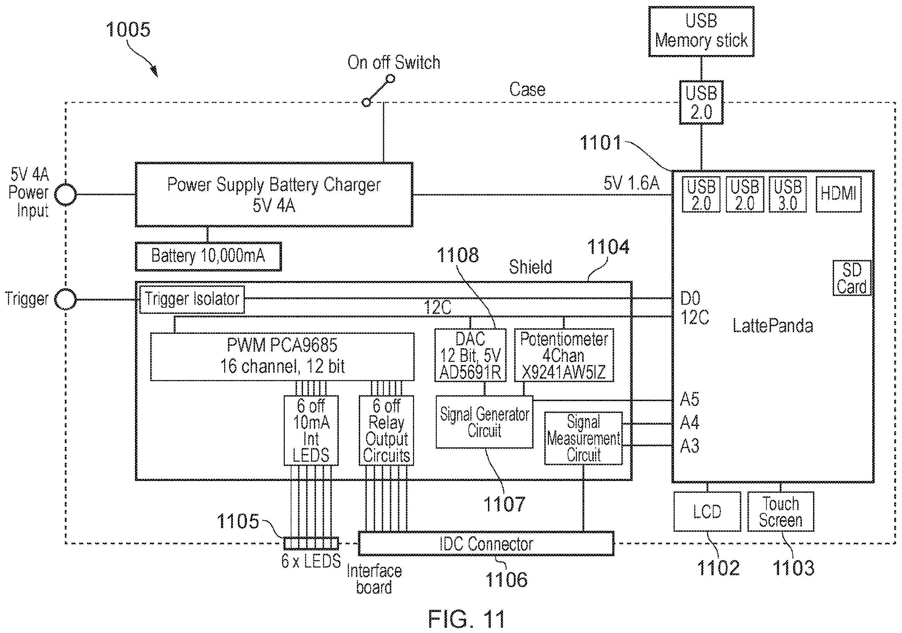

Figure 11 illustrates an example layout of the controller 1005 based around a Lattepanda minicomputer 1101. The computer 1101 interfaces via an LCD screen output 1102 and touch screen input 1103. An interface board 1104 connects the computer 1101 to a light source 1105, which in this example is provided by multiple (e.g. six) LEDs, and to the sample slide via a connector 1106. A signal generator circuit 1107 provides a driving signal to a DAC 1108, which outputs signals to the light source 1105 and selectively to one or more electrodes of the sample slide.

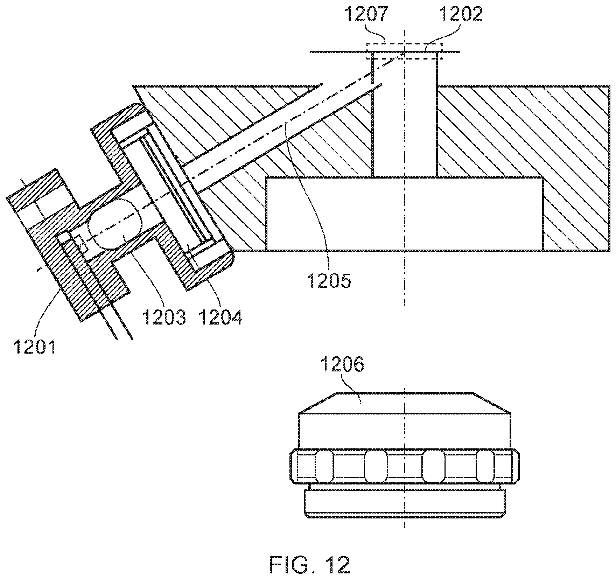

A detailed view of an example optical assembly for illuminating a sample is shown in figure 12. A light source 1201, for example an LED, provides incident light for the sample 1202, the light passing through a lens 1203, filter 1204 and light pipe 1205 before reaching the sample 1202. Light from the sample is received via a lens assembly 1206, which focuses incident light for the image sensor described above. **In** a general aspect, the test volume of the sample comprising cells and a fluorescent dye is illuminated by light from a light source 1201 that is incident upon the sample at an oblique angle to a plane of the sample holder. The incident light may be directed on to the sample via an optical guide 1205. An advantage of this arrangement is that any stray light from the light source 1201 incident on the lens assembly is reduced or minimised.

Figure 13 illustrates an example electrode layout for a sample slide. Three regions 1301a-c are provided on the layout for depositing samples. Each region comprises an interdigitated electrode array, a detailed view of which is shown in figure 14. The spacing in the electrode array may for example be around 0.05 mm, with each electrode track being around 0.07 mm wide.

Other embodiments are intentionally within the scope of the invention as defined by the appended claims.

## Claims

1. A method of detecting vitality of cells in a sample, comprising:
providing a sample (304) comprising cells and a fluorescent dye that responds to a change in membrane polarization;
disposing a portion of the sample (304) containing a medium within which is provided the cells and the fluorescent dye in a test volume between a pair of electrodes (301, 302);
applying a voltage across the pair of electrodes (301, 302) to generate an electric field across the portion of the sample (304);
illuminating the test volume;
measuring a fluorescence response from the test volume over a period of time after applying the voltage across the pair of electrodes (301, 302); and
detecting cells in the sample to be vital dependent on a change in their fluorescence response over the period of time.

2. The method of claim 1 wherein the change in fluorescence is an increase in fluorescence response over the period of time.

3. The method of claim 1 or claim 2 wherein the electric field is less than 1kV/cm and optionally greater than 100V/cm.

4. The method of any preceding claim wherein the electric field is alternating.

5. The method of any preceding claim wherein the voltage is applied for less than 10s or 5s and optionally for greater than 0.1s.

6. The method of any preceding claim comprising the step of detecting cells in the sample to be non-vital cells if their fluorescence response decreases over the period of time.

7. The method of any preceding claim wherein the period of time is less than 60s or less than 10s and/or greater than 0.1s.

8. The method of any preceding claim wherein the fluorescent dye is a Nernstian dye such as a flavin.

9. The method of any preceding claim wherein the sample (304) flows through the test volume from a first location to a second location of the test volume over the period of time, the steps of illuminating the test volume with light and measuring a fluorescent response being repeated at the first and second locations.

10. The method of any preceding claim wherein the step of detecting comprises detecting cells in the sample to be vital if their fluorescence response changes by more than a predetermined amount over the period of time, wherein the predetermined amount is optionally greater than 50%.

11. A method of detecting a strain of bacteria in a sample, comprising:
treating the sample with an antibiotic; and
performing the method of any preceding claim to detect bacteria resistant to the antibiotic.

12. The method of claim 11 wherein the bacteria is *Escherichia coli* or *Bacillus subtilis.*

13. The method of claim 12 wherein the antibiotic is vancomycin.

## Patentansprüche

1. Verfahren zum Nachweis der Zellenvitalität in einer Probe, umfassend:
Bereitstellen einer Probe (304), die Zellen und einen Fluoreszenzfarbstoff umfasst, der auf eine Änderung der Membranpolarisation reagiert;
Anordnen eines Abschnitts der Probe (304), die die Zellen und ein flüssiges oder gelartiges Medium enthält, das den Fluoreszenzfarbstoff enthält, in einem Testvolumen zwischen einem Elektrodenpaar (301, 302);
Anlegen einer Spannung über das Elektrodenpaar (301, 302), um ein elektrisches Feld über dem Abschnitt der Probe (304) zu erzeugen;
Beleuchten des Testvolumens;
Messen einer Fluoreszenzreaktion aus dem Testvolumen über einen Zeitraum nach Anlegen der Spannung über das Elektrodenpaar (301, 302); und
Nachweisen von Zellen in der Probe als vital, abhängig von einer Veränderung ihrer Fluoreszenzreaktion über den Zeitraum.

2. Verfahren nach Anspruch 1, wobei die Änderung der Fluoreszenz eine Zunahme der Fluoreszenzreaktion über den Zeitraum ist.

3. Verfahren nach Anspruch 1 oder Anspruch 2, wobei das elektrische Feld weniger als 1 kV/cm und optional mehr als 100 V/cm beträgt.

4. Verfahren nach einem der vorstehenden Ansprüche, wobei das elektrische Feld ein Wechselstromfeld ist.

5. Verfahren nach einem der vorstehenden Ansprüche, bei dem die Spannung für weniger als 10 s oder 5 s und optional für mehr als 0,1 s angelegt wird.

6. Verfahren nach einem der vorstehenden Ansprüche, umfassend den Schritt des Nachweisens von Zellen in der Probe als nichtvitale Zellen, wenn ihre Fluoreszenzreaktion über einen bestimmten Zeitraum abnimmt.

7. Verfahren nach einem der vorstehenden Ansprüche, wobei der Zeitraum weniger als 60 s oder weniger als 10 s und/oder mehr als 0,1 s beträgt.

8. Verfahren nach einem der vorstehenden Ansprüche, wobei der Fluoreszenzfarbstoff ein Nernstscher Farbstoff wie beispielsweise ein Flavin ist.

9. Verfahren nach einem der vorstehenden Ansprüche, wobei die Probe (304) über den Zeitraum hinweg von einer ersten Stelle zu einer zweiten Stelle des Testvolumens durch das Testvolumen fließt,
wobei die Schritte des Beleuchtens des Testvolumens mit Licht und des Messens einer Fluoreszenzreaktion an der ersten und zweiten Stelle wiederholt werden.

10. Verfahren nach einem der vorstehenden Ansprüche, wobei der Schritt des Nachweisens das Nachweisen vitaler Zellen in der Probe umfasst, wenn sich deren Fluoreszenzreaktion über den Zeitraum um mehr als einen vorbestimmten Wert ändert, wobei der vorbestimmte Wert optional größer als 50 % ist.

11. Verfahren zum Nachweisen eines Bakterienstamms in einer Probe, umfassend:
Behandeln der Probe mit einem Antibiotikum; und
Durchführen des Verfahrens nach einem der vorstehenden Ansprüche zum Nachweis von Bakterien, die gegen das
Antibiotikum resistent sind.

12. Verfahren nach Anspruch 11, wobei das Bakterium Escherichia coli oder Bacillus subtilis ist.

13. Verfahren nach Anspruch 12, wobei das Antibiotikum Vancomycin ist.

## Revendications

1. Procédé de détection de la vitalité de cellules dans un échantillon, comprenant :
le fait de se mettre à disposition un échantillon (304) comprenant des cellules et un colorant fluorescent qui réagit à un changement de polarisation membranaire ;
le fait de disposer une partie de l'échantillon (304) contenant les cellules et un milieu liquide ou gélifié contenant le colorant fluorescent dans un volume d'essai entre une paire d'électrodes (301, 302) ;
le fait d'appliquer une tension entre la paire d'électrodes (301, 302) pour générer un champ électrique à travers la partie de l'échantillon (304) ;
le fait d'éclairer le volume d'essai ;
le fait de mesurer une réponse fluorescente provenant du volume d'essai pendant une période de temps après avoir appliqué la tension entre la paire d'électrodes (301, 302) ; et
le fait de détecter les cellules de l'échantillon qui sont viables en fonction d'un changement dans leur réponse fluorescente pendant la période de temps.

2. Procédé selon la revendication 1, dans lequel le changement de fluorescence est une augmentation de la réponse fluorescente pendant la période de temps.

3. Procédé selon la revendication 1 ou la revendication 2, dans lequel le champ électrique est inférieur à 1 kV/cm et optionnellement supérieur à 100 V/cm.

4. Procédé selon l'une quelconque des revendications précédentes, dans lequel le champ électrique est alternatif.

5. Procédé selon l'une quelconque des revendications précédentes, dans lequel la tension est appliquée pendant moins de 10 s ou 5 s, et optionnellement pendant plus de 0,1 s.

6. Procédé selon l'une quelconque des revendications précédentes, comprenant l'étape consistant à détecter les cellules de l'échantillon comme étant des cellules non viables si leur réponse fluorescente diminue au cours de la période de temps.

7. Procédé selon l'une quelconque des revendications précédentes, dans lequel la période de temps est inférieure à 60 s ou inférieure à 10 s et/ou supérieure à 0,1 s.

8. Procédé selon l'une quelconque des revendications précédentes, dans lequel le colorant fluorescent est un colorant de Nernst, tel qu'une flavine.

9. Procédé selon l'une quelconque des revendications précédentes, dans lequel l'échantillon (304) s'écoule à travers le volume d'essai d'un premier emplacement à un deuxième emplacement du volume d'essai pendant la période de temps, les étapes consistant à éclairer le volume d'essai avec de la lumière et à mesurer une réponse fluorescente étant répétées aux premier et deuxième emplacements.

10. Procédé selon l'une quelconque des revendications précédentes, dans lequel l'étape de détection comprend le fait de détecter des cellules vitales dans l'échantillon si leur réponse fluorescente change de plus d'une quantité prédéterminée pendant la période de temps, dans lequel la quantité prédéterminée est optionnellement supérieure à 50 %.

11. Procédé de détection d'une souche de bactéries dans un échantillon, comprenant :
le fait de traiter l'échantillon avec un antibiotique ; et
le fait de mettre en œuvre le procédé de l'une quelconque des revendications précédentes pour détecter les bactéries résistantes à l'
antibiotique.

12. Procédé selon la revendication 11, dans lequel la bactérie est Escherichia coli ou Bacillus subtilis.

13. Procédé selon la revendication 12, dans lequel l'antibiotique est la vancomycine.
